(19) Europäisches Patentamt
European Patent Office
Office européen des brevets

(11) Numéro de publication: **0 046 001**
**B1**

(12) **FASCICULE DE BREVET EUROPEEN**

(45) Date de publication du fascicule du brevet: **08.08.84**

(21) Numéro de dépôt: **81201106.2**

(22) Date de dépôt: **27.06.79**

(60) Numéro de publication de la demande initiale en application de l'article 76 CBE: **0007823**

(51) Int. Cl.³: **C 07 J 9/00,** C 07 J 21/00, C 07 B 23/00 //A61K31/575, A61K49/02

# (54) Nouveaux dérivés 3-oxo ou 3-cétal, 20-céto diène stéroides, leur procédé de préparation et leur application à la préparation de médicaments.

(30) Priorité: **13.07.78 FR 7820973**

(43) Date de publication de la demande:
**17.02.82 Bulletin 82/7**

(45) Mention de la délivrance du brevet:
**08.08.84 Bulletin 84/32**

(84) Etats contractants désignés:
**AT BE CH DE FR GB IT NL SE**

(56) Documents cités:
**FR - A - 2 149 302**
**FR - A - 2 374 335**
**FR - A - 2 385 737**

(73) Titulaire: **ROUSSEL-UCLAF**
**35, boulevard des Invalides**
**F-75007 Paris (FR)**

(72) Inventeur: **Coussediere, Daniel**
**134, rue de la Fontaine Jean Valjean**
**F-93370 Montfermeil (FR)**

(74) Mandataire: **Tonnellier, Marie-José et al,**
**ROUSSEL-UCLAF 111, route de Noisy Boîte Postale no.9**
**F-93230 Romainville (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

**EP 0 046 001 B1**

Courier Press, Leamington Spa, England.

**Description**

La présente invention concerne de nouveaux dérivés 3-oxo ou 3-cétal, 20-céto diène stéroïdes, leur procédé de préparation et leur application à la préparation de médicaments.

L'invention a pour objet les composés suivants:

A — les composés de formule III:

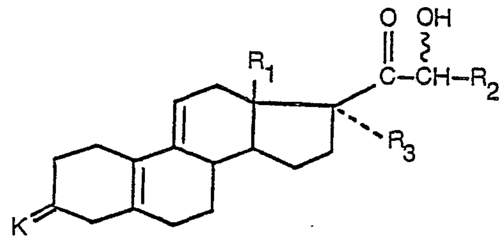

(III)

B — les composés de formule IV:

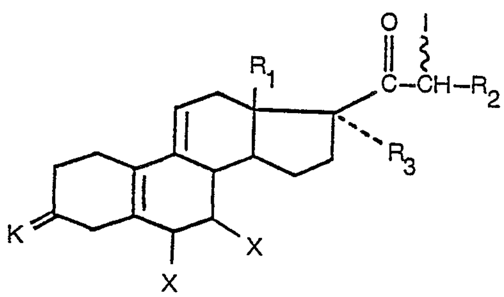

(IV)

C — les composés de formule V:

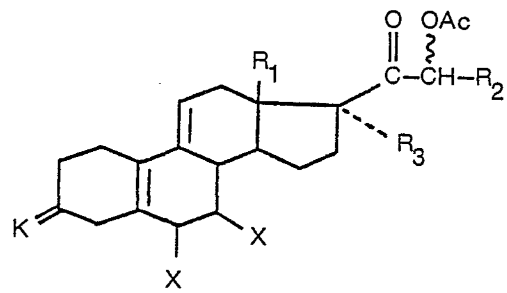

(V)

D — les composés de formule VI:

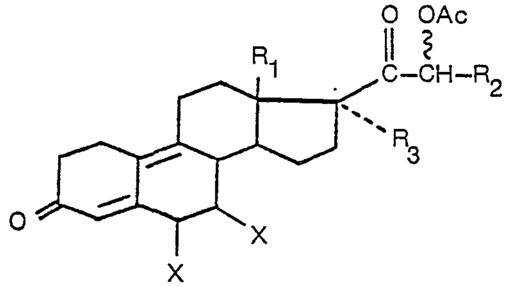

(VI)

formules dans lesquelles K représente un groupement cétal

X représente un atome d'hydrogène ou un atome de tritium.

$R_1$ représente un radical alcoyle renfermant de 1 à 3 atomes de carbone.

$R_2$ un radical alcoyle renfermant de 1 à 12 atomes de carbone.

$R_3$ un radical alcoyle renfermant de 1 à 4 atomes de carbone, sous forme de chacun des épimères 21R ou 21S ou sous forme d'un mélange de ces épimères.

Parmi les composés de formules IV, V ou VI, on peut citer, en particulier, ceux pour lesquels X représente un atome d'hydrogène.

$R_1$ représente, de préférence, le radical méthyle ou éthyle.

$R_2$ représente, de préférence, la radical méthyle, éthyle, n-propyle, isopropyle, n-butyle, isobutyle, n-pentyle, n-hexyle, 2-méthylpentyle, 2,3-diméthylbutyle, n-octyle, ou 2,2-diméthylhexyle.

$R_3$ représente, de préférence, le radical méthyl, éthyle ou n-propyle.

L'invention a tout particulièrement pour objet le composé de formule III qui répond à la dénomination 3-(1,2-éthanediyl) acétal cyclique de 17$\alpha$-méthyl 17$\beta$-(2-hydroxy 1-oxopropyl) estra-5(10), 9 (11) dièn-3-one sous forme de chacun des épimères au niveau de l'hydroxyle ou sous forme d'un mélange de ces épimères, les composés de formule IV qui répondent aux dénominations 3-(1,2-éthanediyl) acétal cyclique de 17$\alpha$-méthyl 17$\beta$-(2-iodo 1-oxopropyl) estra 5 (10), 9 (11)-dièn 3-one et /6,7$^3$H/ 3-(1,2-éthanediyl) acétal cyclique de 17$\alpha$-méthyl 17$\beta$-(2-iodo 1-oxopropyl) estra 5 (10), 9 (11)-dièn 3-one sous forme de chacun des épimères au niveau de l'iode ou sous forme d'un mélange de ces épimères, les composés de formule V qui répondent aux dénominations 3-(1,2-éthanediyl) acétal cyclique de 17$\alpha$-méthyl 17$\beta$-(2-acétoxy 1-oxopropyl) estra 5 (10) 9 (11)-dièn 3-one et /6,7$^3$H/ 3-(1,2-éthanediyl) acétal cyclique de 17$\beta$-(2-acétoxy 1-oxopropyl) 17$\alpha$-méthyl estra 5 (10) 9 (11) dièn 3-one, sous forme de chacun des épimères au niveau de l'acétoxyle ou sous forme d'un mélange de ces épimères, et les composés de formule VI, qui répondent aux dénominations 17$\beta$-(2-acétoxy 1-oxopropyl) 17$\alpha$-méthyl estra-4,9-dièn 3-one et /6,7$^3$H/ 17$\beta$-(2-acétoxy 1-oxopropyl) 17$\alpha$-méthyl estra 4,9-dièn 3-one, sous forme de chacun des épimères au niveau de l'acétoxyle ou sous forme d'un mélange de ces épimères.

L'invention a également pour object un procédé de préparation des composés de formule III caractérisé en ce que l'on soumet un composé de formule II:

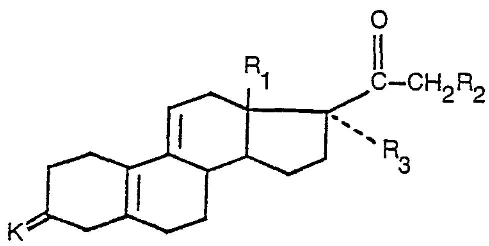

(II)

dans laquelle:

K représente un groupement cétal,

$R_1$, $R_2$ et $R_3$ conservant la même signification que précédemment, à l'action d'un agent d'oxydation en présence d'un alcoolate tertiaire, puis à celle d'un agent de réduction pour obtenir le composé de formule III:

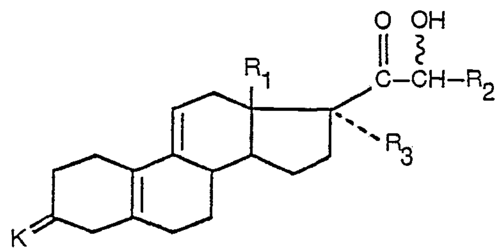

(III)

sous forme d'un mélange d'épimères 21R et 21S.

K représente de préférence un groupement alkyl-cétal cyclique ayant de 2 à 4 atomes de carbone et, notamment, l'éthylènecétal ou le propylènecétal ou bien un dialkylcétal comme, par exemple, le diméthyl ou le diéthylcétal.

L'agent d'oxydation est, de préférence, l'oxygène moléculaire.

L'alcoolate tertiaire est, de préférence, un terbutylate ou un teramylate de métal alcalin, comme par exemple, le terbutylate ou le teramylate de sodium, de potassium ou de lithium.

L'agent de réduction utilisé est, de préférence, un trialcoyl-phosphite, par exemple, le triméthyl- ou le triéthyl phosphite.

L'invention a également pour object un procédé de préparation des composes de formules IV, V et VI, caractérisé en ce que l'on soumet un composé de formule $II_A$:

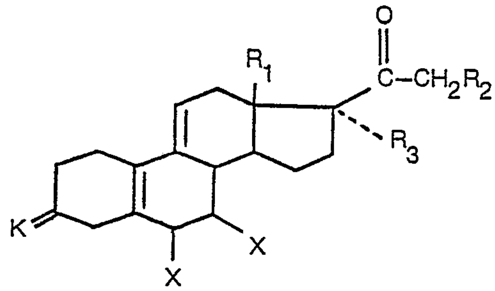

($II_A$)

dans laquelle K, $R_1$, $R_2$, $R_3$ et X ont la signification déjá indiquée, à l'action d'une base forte pour former l'énolate intermédiaire que l'on soumet à l'action de l'iode pour obtenir le composé de formule IV:

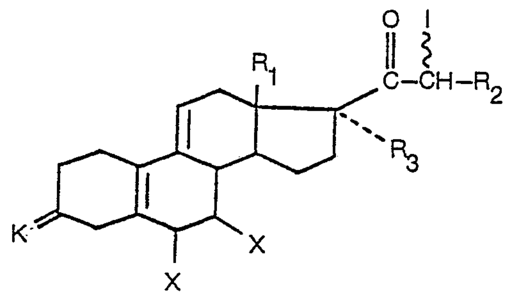

(IV)

sous forme d'un mélange d'isomères 21 R et 21 S, que l'on soumet à l'action d'un acétate alcalin, pour obtenir le composé de formule V:

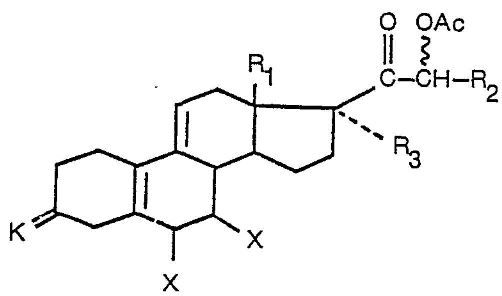

(V)

sous forme d'un mélange d'isomères 21 R et 21 S, que l'on soumet à l'action d'un agent d'hydrolyse acide capable d'hydrolyser le groupement cétal et d'isomériser le système de doubles liaisons △ 5 (10) 9 (11) en un système △ 4,9, pour obtenir le composé de formule VI:

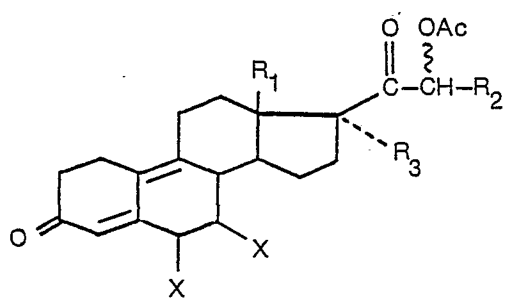

(VI)

sous forme d'un mélange d'isomères 21R et 21S.

La base forte est, de préférence, le butyl lithium et l'on opère en présence d'un amine qui est notamment la N-cyclohexyl isopropylamine. La base forte utilisée peut également être un hydrure alcalin ou un terbutylate alcalin.

L'acétate alcalin est, de préférence, l'acétate de sodium ou de potassium.

L'agent d'hydrolyse acide, capable d'hydrolyser le groupement cétal et d'isomériser le système de doubles liaisons △ 5 (10) 9 (11) en un système △ 4,9 est, de préférence, une résine sulfonique de commerce, à support de polystyrène ou à support de polymère styrène/divinyl-benzène, mais on peut également utiliser un acide minéral tel que l'acide chlorhydrique ou l'acide sulfurique; on peut également utiliser l'acide paratoluène sulfonique ou l'acide perchlorique.

L'invention a également pour objet une application des composés de formule III à la préparation des composés de formule I:

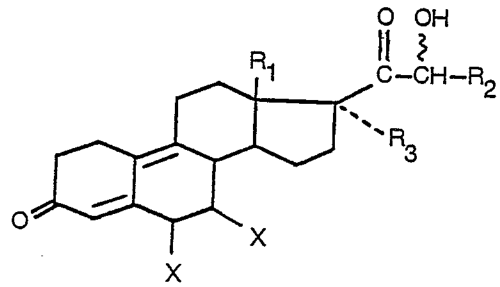

(I)

dans laquelle $R_1$, $R_2$ et $R_3$ ont la signification indiquée précédemment et X représente un atome d'hydrogène, caractérisé en ce que l'on soumet le composé de formule III à l'action d'un agent d'hydrolyse acide capable d'hydrolyser le groupement cétal et d'isomériser le système de doubles liaisons 5 (10) 9 (11) en un système △ 4,9 pour obtenir le composé de formule I dans laquelle X représente un atome d'hydrogène sous forme d'un mélange d'épimères 21R et 21S que l'on sépare, si désiré, en chacun des épimères.

L'agent d'hydrolyse acide capable d'hydrolyser le groupement cétal et d'isomériser le système de doubles liasions △ 5 (10) 9 (11) en un system △ 4,9 peut être l'un de ceux qui ont été cités précédemment.

Les épimères obtenus peuvent, comme ci-dessus, être séparrés par les méthodes classiques de chromatographie.

L'invention a enfin pour objet une application des composés de formule VI à la préparation des composés de formule $I_A$:

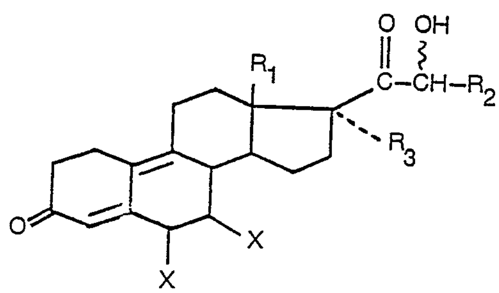

($I_A$)

dans laquelle X, $R_1$, $R_2$ et $R_5$ ont la signification indiquée ci-dessus, caractérisée en ce que l'on soumet un composé de formule VI à l'action d'un agent de saponification, pour obtenir le composé de formule $I_A$ sous forme d'un mélange d'isomères 21R et 21S, que l'on sépare, si désiré, en chacun des épimères.

L'agent de saponification peut être notamment une base alcaline comme la soude ou la potasse.

Les épimères obtenus peuvent, comme ci-dessus, être séparés par les méthodes classiques de chromatographie.

Les composés de formule I obtenus à partir des intermédiaires objet de la présente invention et pour lesquels X représente un atome d'hydrogène présentent d'intéressantes propriétés pharmacologiques, notamment, une activité progestomimétique et une activité anit-estrogène remarquables, comme le montrent les résultats de tests exposés ci-après dans la partie expérimentale. Ces propriétés rendent lesdits produits de formule I aptes à être utilisés comme médicaments progestatifs inhibiteurs hypophysaires à prédominance anti-LH ou comme anti-estrogènes. Ils trouvent leur emploi dans le traitement des dysménorrhées, des stérilités, des dystrophies ovariennes par mise au repos des ovaires, dans le traitement des tumeurs du sein et de l'utérus, et comme contraceptifs.

Les composés de formule I, pour lesquels X représente un atome de tritium possèdent, de manière générale, les mêmes propriétés pharmacologiques que les produits de formule I pour lesquels X représente un atome d'hydrogène.

De plus, ces composés et notamment l'isomère S en 21 décrit ci-après dans la partie expérimentale, en raison de leur activité spécifique, permettent la localisation et le dosage aisé du récapteur spécifique de la progestérone, notamment dans le cytosol utérin ou dans le cytoplasme de cellules tumorales (cancer du sein) et dans les tumeurs induites par le DMBA (le 9,10-diméthyl 1,2-benzanthracène) chez le rat.

Les composés de formule II utilisés comme produits de départ du procédé de l'invention, sont des produits connus d'une façon générale, ils peuvent être préparés par exemple selon le procédé du brevet français 2 149 302.

Les composés de formule $II_A$ dans laquelle X représente un atome de tritium peuvent être préparés selon le procédé de la demande française publiée n° 2 374 335.

Les exemples suivants illustrent l'invention sans toutefois la limiter.

Exemple 1

17α-méthyl 17β-(2-hydroxy 1-oxo propyl) estra 4,9 dien 3-one.

*Stade A: 3-(1,2-éthanediyl) acétal cyclique de 17α-méthyl 17β-(2-hydroxy 1-oxopropyl) estra-5 (10), 9 (11)-dien 3-one.*

On fait barboter de l'oxygène à —20°C. dans une solution renfermant 907 mg de terbutylate de potassium et 0,852 cm3 de triéthylphosphite dans 13,6 cm3 de diméthylformamide. On ajoute lentement, à —20°C., une solution renfermant 1 g de 3-(1,2-éthanediyl) acétal cyclique de 17α-méthyl 17β-(1-oxopropyl) estra 5 (10), 9 (11) dien 3-one dans 10 cm3 de diméthylformamide en maintenant toujours le barbotage d'oxygène. On laisse 30 minutes à —20°C. puis l'on ajoute de l'eau et amène à pH 5,6 par addition d'acide chlorhydrique N. On extrait cinq fois par 150 cm3 d'éther isopropylique. On sèche les phases organiques, les filtre et concentre à sec. On obtient ainsi 1,554 g de produit recherché que l'on utilise tel quel dans le stade suivant.

*Stade B: 17α-méthyl 17β-(2-hydroxy 1-oxo propyl) estra 4,9 dien 3-one*

On ajoute 750 mg d'une résine REDEX CF dans 3 cm3 d'éthanol à 95° renfermant 510 mg du produit obtenu au stade A. On chauffe au reflux le mélange obtenu pendant 7 heures. On filtre, rince la résine obtenue à l'éthanol et au chlorure de méthylène. On concentre à sec sous pression réduite. On obtient 474 mg d'une résine que l'on chromatographie sur silice:
(éluant: chlorure de méthylène/acétone 9—1). On isole la fraction contenant les 2 épimères en 21. Ces deux épimères sont séparés par chromatographie liquide sous haute pression:
colonnes de silice (silices 5μ) de 6 mm de diamètre interne et de 400 mm de longeur.
Débit: 160 ml à l'heure.
Solvant: chlorure de méthylène/acétate d'éthyle (7—3)
Détecteur U.V. = 305 nm.
Le produit dont le temps de rétention est 19 minutes est appelé A.
Celui dont le temps de rétention est 17 minutes est appelé B.
Les constantes physiques du produit A sont les suivantes:

a) *Spectre R.M.N.* ($CDCl_3$)
$H_4$ éthylénique à 5,70 ppm
$CH_{3\ 13}$ à 0,84 ppm
$H_{21}$ à 4,33 ppm multiplets
$CH_3$ 22 doublet à 1,27 ppm et 1,38 ppm
$CH_{3\ 17\alpha}$ 1,18 ppm.

b) *Spectre de masse : pic moléculaire à 342*

Les constantes physiques du produit B sont les suivantes:

a) *Spectre R.M.N.* ($CDCl_3$)
$H_4$ éthylénique à 5,70 ppm
$CH_{3\ 13}$ 0,81 ppm
$H_{21}$ à 4,38 ppm, 4,50 ppm, 4,61 ppm, 4,73 ppm
$CH_{3\ 22}$ 1,26 ppm, 1,37 ppm
$CH_{3\ 17\alpha}$ 1,16 ppm

b) *Spectre de masse pic moléculaire à 342*

Exemple 2

17β-(2-hydroxy 1-oxopropyl) 17α-méthyl estra 4,9-dièn 3-one (isomère A et isomère B)

*Stade A: 3-(1,2-éthanediyl) acétal cyclique de 17α-méthyl 17β-(2-iodo 1-oxopropyl) estra 5 (10) 9 (11)-dièn 3-one (mélange d'isomères)*

a) Formation de l'énolate

On met en solution, sous atmosphère inerte, 11,1 cm3 de N-cyclohexyl isopropylamine dans 145 cm3 de tétrahydrofuranne, refroidit à —40°C, adjoute 23,3 cm3 de butyllithium dans l'hexane, agite 5 minutes et ajoute 10,3 g de 3-(1,2-éthanediyl) acétal cyclique de 17α-méthyl 17β-(1-oxopropyl) estra 5 (10), 9 (11)-dièn 3-one en agitant encore à 40°C pendant 20 minutes.

b) Formation du dérivé iodé

On refroidit à —50°C une solution de 8,2 g d'iode dans 60 cm3 de tétrahydrofuranne, ajoute en 5 minutes la solution d'énolate précédente, agite 5 minutes à —50°C et laisse la solution revenir à température ambiante. On verse celle-ci sur 100 cm3 de solution saturée de chlorure d'ammonium dans l'eau, extrait à l'éther, lave avec une solution à 10% de thio sulfate de sodium dans l'eau, puis à l'eau, sèche et distille à sec sous pression réduite pour obtenir 14,5 g de produit utilisé tel quel et immédiatement pour le stade suivant.

*Stade B: 3-(1,2-éthanediyl) acétal cyclique de 17α-méthyl 17β-(2-acétoxy 1-oxopropyl) estra 5 (10) 9 (11) dièn 3-one (mélange d'isomères)*

On agite à 80°C, sous atmosphère inerte, pendant 1 heure, 14,9 g du dérivé iodé obtenu au Stade A, 29g d'acétate de potassium anhydre et 50 cm3 de diméthylformamide. On verse le mélange sur 300 cm3 d'eau, extrait à l'éther, lave à l'eau, sèche et concentre à sec sous pression réduite. On obtient 11,8 g de produit attendu.

*Stade C: 17β-(2-acétoxy 1-oxopropyl) 17α-méthyl estra 4,9-dièn 3-one (mélange d'isomères).*

On met en solution, sous atmosphère inerte, 11,8 g de produit obtenu ci-dessus, 110 cm3 d'acide acétique et 11 cm3 d'acide perchlorique. On agite 1 heure à température ambiante, dilue avec 400 cm3 d'eau, extrait au chlorure de méthylène, lave avec une solution aqueuse saturée de bicarbonate de sodium, sèche et concentre à sec sous pression réduite. On obtient 12 g de produit que l'on purifie par chromatographie sur silice en éluant par le mélange benzène — acétate d'éthyle (8—2). On recueille 8,6 g de produit utilisé tel quel pour le stade suivant.

*Stade D: 17β-(2-hydroxy 1-oxopropyl) 17α-méthyl estra 4,9 dièn 3-one (isomère A et isomère B).*

On met en solution, sous atmosphère inerte, 8,6 g du produit obtenu précédemment dans 50 cm3 de méthanol, ajoute 86 mg de potasse et agite 5 heures à température ambiante. On ajoute 50 cm3 d'acide chlorhydrique 0,1 N et extrait au chlorure de méthylène, sèche, concentre à sec sous pression réduite et obtient 7,6 g de produit. Par chromatographie sur silice sous pression, en éluant par le mélange benzène — acétate d'éthyle (8—2), on récupère 2,7 g d'isomère B et 2,5 g d'isomère A.

On recristallise l'isomère B dans l'éther, puis dans l'éthanol et obtient 1,3 g de produit fondant à 190°C.

*Analyse:*

| | | | | |
|---|---|---|---|---|
| Calculé: | C% | 77,15 | H% | 8,83 |
| Trouvé: | | 77,2 | | 9,0 |

$/\alpha/_D^{20} = -345° \pm 4.5°$ (c = 1% $CHCl_1$)

*Spectre RMN ($CDCl_3$)*

Hydrogène en 2 du propyle: quintuplet à 4,58 ppm J = 7

Hydrogènes en 3 du propyle: à 1,27—1,38 ppm

Hydrogènes du $CH_3$ en 17α: à 1,17 ppm

Hydrogènes du $CH_3$ en 18: à 0,82 ppm

On recristallise l'isomère A dans l'éther et obtient 1,2 g de produit fondant à 122°C.

*Analyse:*

| | | | | |
|---|---|---|---|---|
| Calculé: | C% | 77,15 | H% | 8,83 |
| Trouvé: | | 77,2 | | 9,1 |

$/\alpha/_D^{20} = -208° \pm 3°$ (c = 1%, $CHCl_3$)

*Spectre RMN ($CDCl_3$)*

Hydrogène en 2 du propyle: quadruplet de 4,2 à 4,67 ppm

Hydrogènes du $CH_3$ en 17α à 1,18 ppm

Hydrogènes du $CH_3$ en 18 à 0,84 ppm

L'analyse par dichroïsme circulaire ainsi que le diagramme de diffraction des rayons X ont permis de déterminer que l'isomère appelé A à la configuration 21 S.

Exemple 3

/6,7$^3$H/ 17$\beta$-(2-hydroxy 1-oxopropyl) 17$\alpha$-méthyl estra 4,9 dièn 3-one (isomère A et isomère B)

*Stade A: /6,7$^3$H/ 3-(1,2-éthanediyl) acétal cyclique de 17$\alpha$-méthyl 17$\beta$-(2-iodo 1-oxopropyl) estra 5 (10) 9 (11) dièn 3-one (mélange d'isomères)*

En opérant de la même manière qu'au Stade A de l'exemple 2 mais en partant de 17,5 mg de /6,7$^3$H/ 3-(1,2-éthanediyl) acétal cyclique de 17$\alpha$-méthyl 17$\beta$-(1-oxopropyl) estra 5 (10) 9 (11)-dièn 3-one, on obtient le produit attendu, sous forme de résine utilisée immédiatement pour le stade suivant.

*Stade B: /6,7$^3$H/ 3-(1,2-éthanediyl) acétal cyclique de 17$\alpha$-méthyl 17$\beta$-(2-acétoxy 1-oxopropyl) estra 5 (10) 9 (11) dièn 3-one (mélange d'isomères)*

Le produit obtenu au stade précédent est traité comme décrit au Stade B de l'exemple 2, en extrayant par de l'acétate d'éthyle à 1‰ de triéthylamine. On obtient le produit attendu qu'on utilise tel quel pour le Stade C.

*Stade C: /6,7$^3$H/ 17$\beta$-(2-acétoxy 1-oxopropyl) 17$\alpha$-méthyl estra 4,9 dièn 3-one (mélange d'isomères)*

En partant du produit obtenu précédemment, on suit la même technique qu'au Stade C de l'exemple 2, pour obtenir une résine qui est utilisée telle quelle au stade suivant.

*Stade D: /6,7$^3$H/ 17$\beta$-(2-hydroxy 1-oxopropyl) 17$\alpha$-*méthyl estra 4,9 dièn 3-one (isomère A et isomère B).

On opère comme au Stade D de l'exemple 2 avec le produit tritié obtenu ci-dessus et sépare 2,64 mg d'isomère B (21 R), puis 1,69 mg d'isomère A (21 S).

La /6,7$^3$H/ 3-(1,2-éthanediyl) acétal cyclique de 17$\alpha$-méthyl 17$\beta$-(1-oxopropyl) estra 5 (10) 9 (11) dièn 3-one utilisée au départ de l'exemple 3 a été préparée de la manière suivante:

On agite, sous atmosphère inerte, 23,8 mg de /6,7$^3$H/ 17$\alpha$-méthyl 17$\beta$-(1-oxo propyl) estra 4,9 dièn 3-one décrit dans la demande française publiée sous le n° 2 374 335, 2 cm3 d'éthylène glycol contenant 1 mg d'acide paratoluène sulfonique et 1 cm3 d'orthoformiate d'éthyle. On chauffe la suspension à 60°C pendant 10 minutes, ajoute de la triéthylamine, refroidit, ajoute une solution aqueuse saturée de bicarbonate de sodium, extrait à l'acétate d'éthyle, lave à l'eau salée, sèche et concentre à sec sous pression réduite. Le produit obtenu est purifié par chromatographie sous pression sur silice en éluant par un mélange benzène — acétate d'éthyle (9—1) contenant 5‰ de triéthylamine.

Etude pharmacologique

*I — Activité progestomimétrique du produit A*

a) L'activité progestomimétique du produit A a été étudiée par la méthode des récepteurs hormonaux décrite par J. P. RAYNAUD et Coll. dans "J. Ster. BIOCHEM" 1975 *6*, 615—622 et dans "Physiology and Genetics of Reprodution 1975 *part A* 143—160".

*La technique est la suivante:*

On administre à des lapines impubères 25 $\mu$g d'estradiol par voie per-cutanée. Cinq jours après ce traitement, les animaux sont sacrifiés, les utérus sont alors prélevés et homogénéisés dans un tampon trométhamine 10 mM, saccharose 0,25 M, HCl.ph 7,4. L'homogénat est centrifugé à 105 000 g pendant une heure. Le liquide surnageant ou cytosol est alors ajusté de façon à avoir une dilution de 1/50 (poids/volume).

On incube à 0°C pendant deux heures des tubes de même volume de cytosol avec une concentration fixe de 17,21-diméthyl 19-nor-4,9-pregnadiène 3,20-dione tritié, désigné ci-après par produit R tritié en présence ou non d'une concentration croissante de 17,21-diméthyl 19-nor-4,9-pregnadiène 3,20-dione radio-inerte désignée ci-après produit R froid, de progestérone ou de produit à tester.

On détermine au bout de deux heures et au bout de 24 heures la radioactivité du produit R tritié lié par la technique d'adsorption au charbon-dextran (1,25%—0,625%).

On trace ensuite:

— la droite parallèle à l'axe des abscisses, d'ordonnée

$$I_{50} = \frac{100\left(1 + \frac{Bmin}{Bo}\right)}{2}$$

Bo étant la quantité maximum de produit R tritié lié, mesurée dans l'incubat contenant uniquement du produit R tritié.

Bmin étant la quantité minimum de produit R tritié lié (non spécifique), mesurée dans l'incubat contenant du produit R tritié plus un grand excés de produit R froid (2500 $10^{-9}$M).

— les courbes représentant les pourcentages de produit R tritié lié

$$\frac{B}{Bo}$$

en fonction du logarithme des concentrations du produit froid ajouté.

Les intersections de la droite $I_{50}$ et des courbes permettent de déterminer les valeurs CP et CX.

CP: concentration de la progestérone froide qui inhibe de 50% la fixation du produit R tritié.

CX: concentration du produit testé qui inhibe de 50% la fixation du produit R tritié.

L'affinité relative du produit testé ou ARL est donnée par la formule:

$$ARL = 100 \times \frac{CP}{CX}$$

RESULTATS:

| PRODUIT | ARL 2 heures | ARL 24 heures |
|---|---|---|
| Progesterone | 100 | 100 |
| Produit A | 204 | 688 |

CONCLUSION: Le produit A présente une affinité pour le récepteur utérin spécifique de la progesterone très supérieure à celle de la progesterone. Le produit A présente donc une très forte activité progestomimétique.

b) L'activité progestomimétique a été déterminée sur le test de CLAUBERG. Selon ce test, des groupes de trois lapines impubères sont préalablement sensibilisées par administration, par voie sous-cutanée, d'estradiol, pendant cinq jours à la dose quotidienne de 5$\mu$g, deux jours plus tard elles sont traitées quotidiennement, pendant cinq jours, avec le médicament étudié, par voie sous-cutanée. Les animaux sont sacrifiés le sixième jour et sur les coupes de l'utérus, la prolifération en dentelle de l'endomètre, caractéristique de l'action progestomimétique, est notée en unités Mac Phail.

Le produit A est utilisé en solution dans l'huile de sésame additionnée de 5% d'alcool benzylique.

| TRAITEMENT par | DOSES $\mu$g/Lapin/Jour | UNITES Mac Phail |
|---|---|---|
| Produit A | 1 | 2,2 |
| | 3 | 3,4 |
| | 10 | 3,4 |

On a donc obtenu 2,2 Unités Mac Phail à la dose quotidienne de 1 $\mu$g. Le produit étudié possède donc une très forte activité progestomimétique.

II — *Détermination de l'activité antiestrogène du Produit A.*

L'activité antiestrogène de ce produit a été recherchée sur la souris impubère selon une technique inspirée du test de RUBIN, Endo. 1951, *49*, 429 et voisine de celle de DORFMAN et Coll. (Methods in Hormone Research. Dorfman, 1962, vol.II.118).

L'estrogène utilisé est l'estradiol. Des groupes de quatre souris âgées de 18 jours reçoivent, en injection sous-cutanée quotidienne, pendant trois jours, soit l'estradiol seul, soit le produit étudié seul, soit l'estradiol et le produit étudié. Dans ce dernier cas, les deux stéroïdes sont injectés en des points différents. Les souris sont sacrifiées le quatrième jour et leur utérus est prélevé et pesé.

L'estradiol en solution dans l'huile de sésame additionnée de 5% d'alcool benzylique, a été administré à la dose totale de 0,27 μg, chaque injection étant pratiquée sous un volume de 0,1 cm3/souris.

Le produit étudié a été utilisé en solution dans l'huile de sésame additionnée de 5% d'alcool benzylique et administré aux doses totales de 0,3—1,3 et 10 μg, les injections étant également practiquées sous un volume de 0,1 cm3/souris.

Les résultats obtenus sont réunis dans le tableau II.

TABLEAU II

| LOTS | DOSE TOTALE (μg) | MOYENNE DES POIDS des utérus en mg. |
|---|---|---|
| Témoins | 0 | 14,4 |
| Estradiol | 0,27 | 99,3 |
| Produit A | 0,3 | 14,6 |
| Produit A + Estradiol | 0,3 + 0,27 | 80,5 −19% |
| Produit A | 1 | 13,0 |
| Produit A + Estradiol | 1 + 0,27 | 59,6 −39% |
| Produit A | 3 | 17,1 |
| Produit A + Estradiol | 3 + 0,27 | 38,5 −61% |
| Produit A | 10 | 15,8 |
| Produit A + Estradiol | 10 + 0,27 | 39,6 −60% |

Ces résultats montrent donc que le produit étudié possède une nette activité antiestrogène vis-à-vis de l'estradiol à la dose de 1 μg.

Exemple de compositions pharmaceutiques

On a préparé des comprimés répondant à la formule suivante:

— produit A (exemple 1) 100 μg

— excipient q.s.pour un comprimé terminé à 100 mg

detail de l'excipient (talc, amidon, stéarate de magnesium).

**Revendications**

1. A — Les composés de formule III:

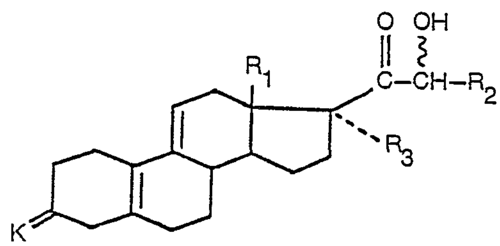

(III)

B — les composés de formule IV:

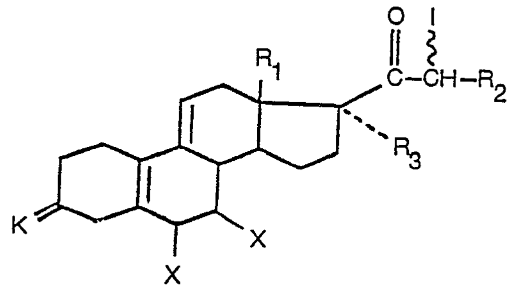

(IV)

C — les composés de formule V:

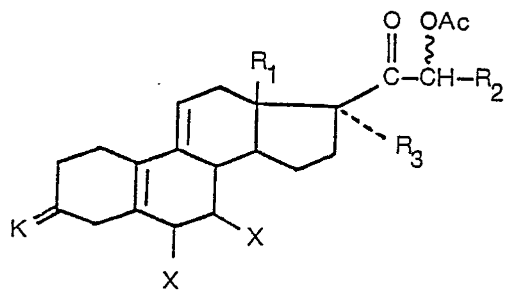

(V)

D — les composés de formule VI:

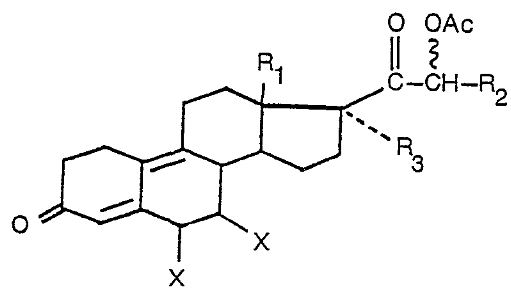

(VI)

formules dans lesquelles K représente un groupement cétal.

X représente un atome d'hydrogène ou un atome de tritium.

$R_1$ représente un radical alcoyle renfermant de 1 à 3 atomes de carbone,

$R_2$ un radical alcoyle renfermant de 1 à 12 atomes de carbone,

$R_3$ un radical alcoyle renfermant de 1 à 4 atomes de carbone, sous forme de chacun des épimères 21R ou 21S ou sous forme d'un mélange de ces épimères.

2. Composé de formule III selon la revendication 1 qui répond à la dénomination 3-(1,2-éthanediyl) acétal cyclique de 17$\alpha$-méthyl 17$\beta$-(2-hydroxy 1-oxopropyl) estra-5 (10), 9 (11) dièn-3-one sous forme de chacun des épimères au niveau de l'hydroxyle ou sous forme d'un mélange de ces épimères.

3. Composés de formule IV selon la revendication 1 qui répondent aux dénominations 3-(1,2-éthanediyl) acétal cyclique de 17$\alpha$-méthyl 17$\beta$-(2-iodo 1-oxopropyl) estra 5 (10), 9 (11)-dièn 3-one et /6,7$^3$H/ 3-(1,2-éthanediyl) acétal cyclique de 17$\alpha$-methyl 17$\beta$-(2-iodo 1-oxopropyl) estra 5 (10), 9 (11)-dién 3-one sous forme de chacun des épimères au niveau de l'iode ou sous forme d'un mélange de ces épimères.

4. Composés de formule V selon la revendication 1 qui répondent aux dénominations 3-(1,2-éthanediyl) acétal cyclique de 17$\alpha$-méthyl 17$\beta$-(2-acétoxy 1-oxopropyl) estra 5 (10) 9 (11)-dièn 3-one et /6,7$^3$H/ 3-(1,2-éthanediyl) acétal cyclique de 17$\beta$-(2-acétoxy 1-oxopropyl) 17$\alpha$-méthyl estra 5 (10) 9 (11) dièn 3-one, sous forme de chacun des épimères au niveau de l'acétoxyle ou sous forme d'un mélange de ces épimères.

5. Composés de formule VI selon la revendication 1 qui répondent aux dénominations 17$\beta$-(2-

acétoxy 1-oxopropyl) 17α-méthyl estra-4,9-dièn 3-one et /6,7[3]H/ 17β-(2-acétoxy 1-oxopropyl) 17α-méthyl estra 4,9-dièn 3-one, sous forme de chacun des épimères au niveau de l'acétoxyle ou sous forme d'un mélange de ces épimères.

6. Procédé de préparation des composés de formule III tels que définis à la revendication 1, caractérisé en ce que l'on soumet un composé de formule II:

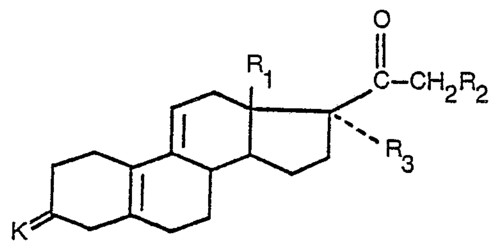

(II)

dans laquelle:

K représente un groupement cétal,

$R_1$, $R_2$ et $R_3$ conservant la même signification que dans la revendication 1, à l'action d'un agent d'oxydation en présence d'un alcoolate tertiaire, puis à celle d'un agent de réduction pour obtenir le composé de formule III sous forme d'un mélange d'épimères 21R et 21S.

7. Procédé de préparation des composés de formule IV, V et V tels que définis à la revendication 1, caractérisé en ce que l'on soumet un composé de formule $II_A$:

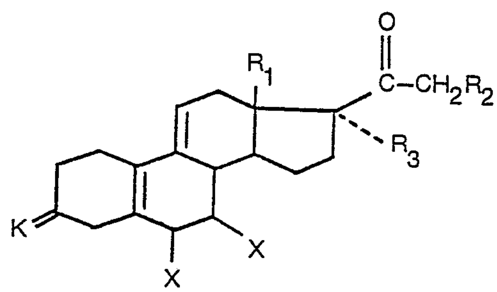

($II_A$)

dans laquelle K, X, $R_1$, $R_2$ et $R_3$ ont la signification indiquée à la revendication 1, à l'action d'une base forte pour former l'énolate intermédiaire que l'on soumet à l'action de l'iode, pour obtenir le composé de formule IV:

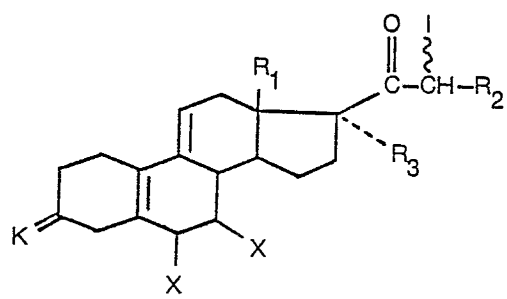

(IV)

sous forme d'un mélange d'isomères 21R et 21S, que l'on soumet, si désiré, à l'action d'un acétate alcalin, pour obtenir le composé de formule V:

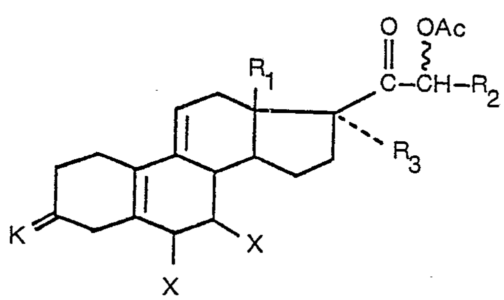

(V)

sous forme d'un mélange d'isomères 21R et 21S, que l'on soumet, si désiré, à l'action d'un agent d'hydrolyse acide capable d'hydrolyser le groupement cétal et d'isomériser le système de doubles liaisons △ 5 (10) 9 (11) en un système 4,9 pour obtenir le composé de formule VI:

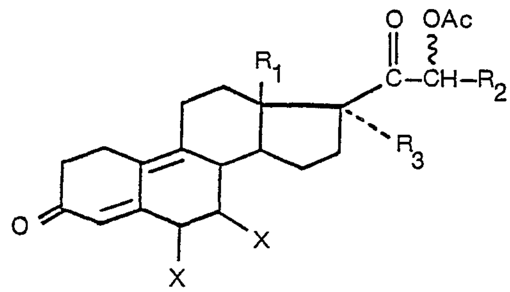

(VI)

sous forme d'un mélange d'isomères 21R et 21S.

8. Application des composés de formule III tels que définis à la revendication 1 à la préparation de composés de formule I:

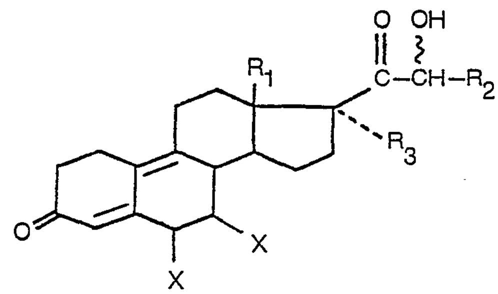

(I)

dans laquelle $R_1$, $R_2$ et $R_3$ ont la signification indiquée à la revendication 1 et X représente un atome d'hydrogène, caractérisé en ce que l'on soumet le composé de formule III à l'action d'un agent d'hydrolyse acide capable d'hydrolyser le groupement cétal et d'isomériser le système de doubles liaisons 5 (10) 9 (11) en un système △ 4,9 pour obtenir le composé de formule I dans laquelle X représente un atome d'hydrogène sous forme d'un mélange d'épimères 21R et 21S l'on sépare, si désiré, en chacun des épimères.

9. Application des composés de formule VI tels que définis à la revendication 1 à la préparation des composés de formule $I_A$:

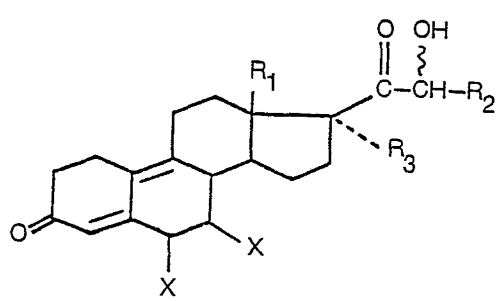

($I_A$)

dans laquelle X, $R_1$, $R_2$ et $R_3$ ont la signification indiquée à la revendication 1, caractérisée en ce que l'on soumet un composé de formule VI à l'action d'un agent de saponification pour obtenir le composé de formule $I_A$ sous forme d'un mélange d'isomères 21R et 21S, que l'on sépare, si désiré, en chacun des épimères.

**Patentansprüche**

1. A. Die Verbindungen der Formel III

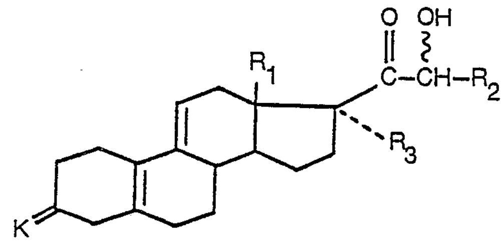

(III)

B. Die Verbindungen der Formel IV

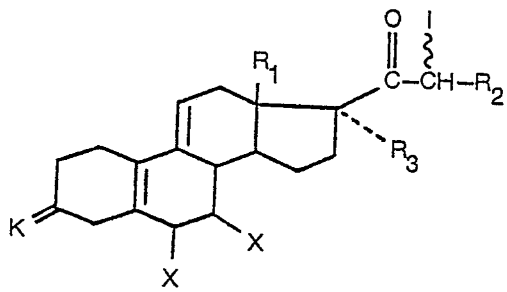

(IV)

C. Die Verbindungen der Formel V

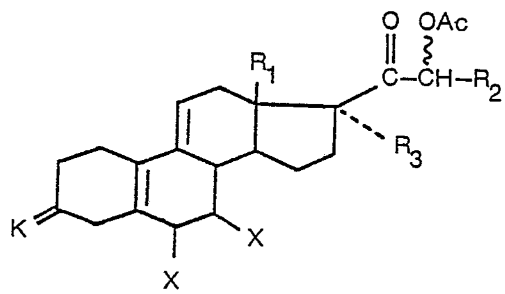

(V)

D. Die Verbindungen der Formel VI

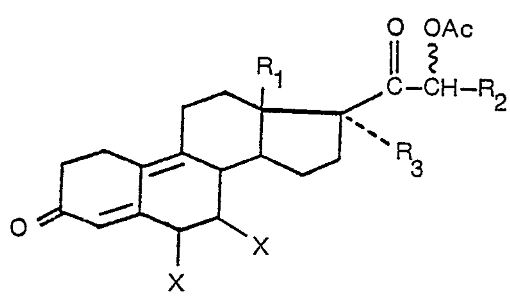

(VI)

worin K eine Ketalgruppe bedeutet,
X ein Wasserstoffatom oder ein Tritiumatom bedeutet,
$R_1$ einen Alkylrest mit 1 bis 3 Kohlenstoffatomen bedeutet,
$R_2$ einen Alkylrest mit 1 bis 12 Kohlenstoffatomen bedeutet,
$R_3$ einen Alkylrest mit 1 bis 4 Kohlenstoffatomen bedeutet,
in Form eines jeden der 21R- oder 21S-Epimeren oder in Form eines Gemisches dieser Epimeren.

2. Verbindungen der Formel III gemäß Anspruch 1 mit der folgenden Bezeichnung: cyclisches 3-(1,2-Äthandiyl)-acetal von 17α-Methyl-17β-(2-hydroxy-1-oxopropyl)-östra-5 (10), 9 (11)-dien-3-on in Form eines jeden der Epimeren im Hinblick auf die Hydroxylgruppe oder in Form eines Gemisches dieser Epimeren.

3. Verbindungen der Formel IV gemäß Anspruch 1, mit den folgenden Bezeichnungen: cyclisches 3-(1,2-Äthandiyl)-acetal von 17α-Methyl-17β-(2-jod-1-oxopropyl)-östra-5 (10), 9 (11)-dien-3-on und cyclisches [6,7$^3$H]-3-(1,2-Äthandiyl)-acetal von 17α-Methyl-17β-(2-jod-1-oxopropyl)-östra-5 (10), 9 (11)-dien-3-on in Form eines jeden der Epimeren im Hinblick auf das Jod oder in Form eines Gemisches dieser Epimeren.

4. Verbindungen der Formel V gemäß Anspruch 1, mit den folgenden Bezeichnungen: cyclisches 3-(1,2-Äthandiyl)-acetal von 17α-Methyl-17β-(2-acetoxy-1-oxopropyl)-östra-5 (10), 9 (11)-dien-3-on und cyclisches [6,7$^3$H]-3-(1,2-Äthandiyl)-acetal von 17β-(2-Acetoxy-1-oxopropyl)-17α-methyl-östra-5 (10), 9 (11)-dien-3-on in Form eines jeden der Epimeren im Hinblick auf das Acetoxyl oder in Form eines Gemisches dieser Epimeren.

5. Verbindungen der Formel VI gemäß Anspruch 1, mit den folgenden Bezeichnungen: 17β-(2-Acetoxy-1-oxopropyl)-17α-methyl-östra-4,9-dien-3-on und [6,7$^3$H]-17β-(2-Acetoxy-1-oxopropyl)-17α-methyl-östra-4,9-dien-3-on in Form eines jeden der Epimeren im Hinblick auf das Acetoxyl oder in Form eines Gemisches dieser Epimeren.

6. Verfahren zur Herstellung der Verbindungen der Formel III gemäß Anspruch 1, dadurch gekennzeichnet, daß man eine Verbindung der Formel II

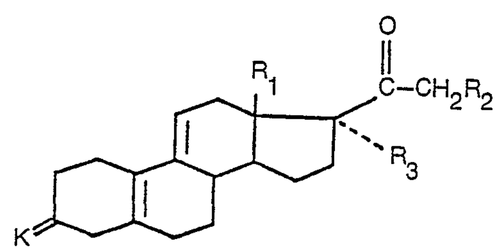

(II)

worin

K eine Ketalgruppe bedeutet,

$R_1$, $R_2$ und $R_3$ die in Anspruch 1 angegebene Bedeutung besitzen,

der Einwirkung eines Oxidationsmittels in Gegenwart eines tertiären Alkoholats und danach derjenigen eines Reduktionsmittels unterzieht, um die Verbindungen der Formel III in Form eines Gemisches der 21R- und 21S-Epimeren zu erhalten.

7. Verfahren zur Herstellung der Verbindung der Formel IV, V und VI gemäß Anspruch 1, dadurch gekennzeichnet, daß man eine Verbindung der Formel $II_A$

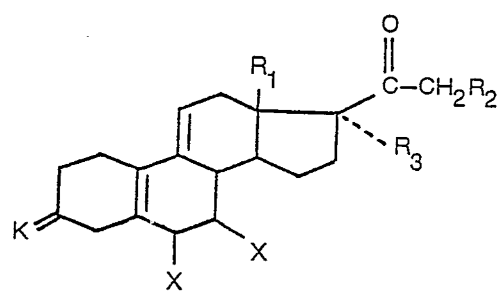

($II_A$)

worin K, X, $R_1$, $R_2$ und $R_3$ die in Anspruch 1 angegebene Bedeutung besitzen, der Einwirkung einer starken Base unterzieht, um hieraus als Zwischenprodukt das Enolat zu bilden, das man der Einwirkung von Jod unterzieht, um die Verbindung der Formel IV

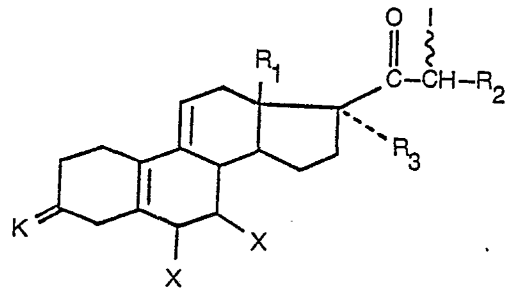

(IV)

in Form eines Gemisches der 21R- und 21S-Isomeren zu erhalten, das man gewünschtenfalls der Einwirkung eines Alkaliacetats unterzieht, um die Verbindung der Formel V

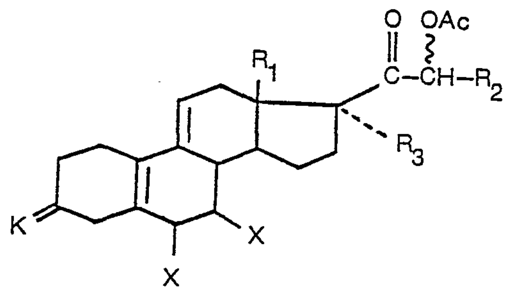

(V)

in Form eines Gemisches der 21R- und 21S-Isomeren zu erhalten, das man gewünschtenfalls der Einwirkung eines sauren Hydrolysemittels unterzieht, das befähigt ist, die Ketalgruppe zu hydrolysieren, und das △ 5 (10), 9 (11)-Doppelbindungs-system in ein △ 4,9-System zu isomerisieren, um die Verbindung der Formel VI

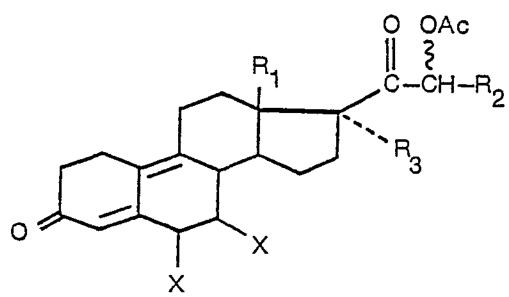

(VI)

in Form eines Gemisches der 21R- und 21S-Isomeren zu erhalten.

8. Verwendung der Verbindungen der Formel III gemäß Anspruch 1 zur Herstellung von Verbindungen der Formel I

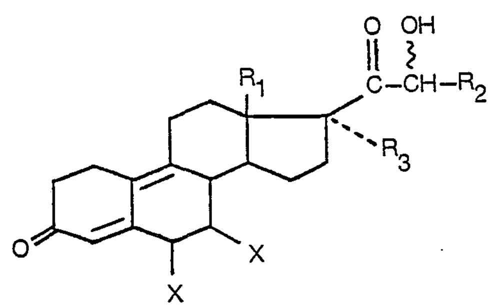

(I)

worin $R_1$, $R_2$ und $R_3$ die in Anspruch 1 angegebene Bedeutung besitzen, und X ein Wasserstoffatom bedeutet, dadurch gekennzeichnet, daß man die Verbindung der Formel III der Einwirkung eines sauren Hydrolysemittels unterzieht, das befähigt ist, die Ketalgruppe zu hydrolysieren, und das △ 5 (10), 9 (11)-Doppelbindungssystem in ein △ 4,9-System zu isomerisieren, um die Verbindung der Formel I, worin X ein Wasserstoffatom bedeutet, in Form eines Gemisches der 21R- und 21S-Epimeren zu

erhalten, das man gewünschtenfalls in ein jedes der Epimeren auftrennt.

9. Verwendung der Verbindungen der Formel VI gemäß Anspruch 1, zur Herstellung von Verbindungen der Formel $I_A$

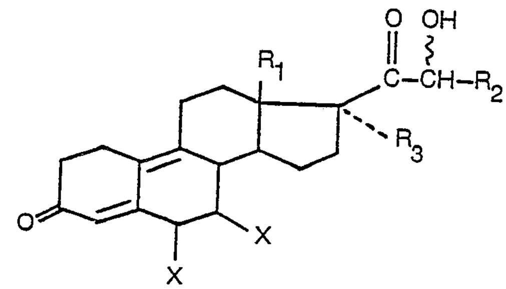

$(I_A)$

worin X, $R_1$, $R_2$ und $R_3$ die in Anspruch 1 angegebene Bedeutung besitzen, dadurch gekennzeichnet, daß man eine Verbindung der Formel VI der Einwirkung eines Verseifungsmittels unterzieht, um die Verbindung der Formel $I_A$ in Form eines Gemisches der 21R- und 21S-Isomeren zu erhalten, das man gewünschtenfalls in ein jedes der Epimeren auftrennt.

**Claims**

1. A — The compounds with the formula (III):

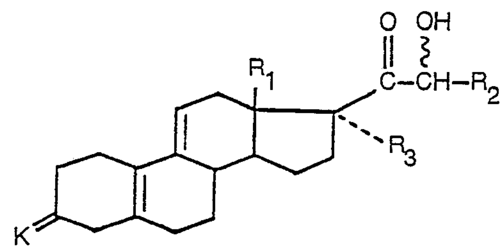

(III)

B — the compounds with the formula (IV):

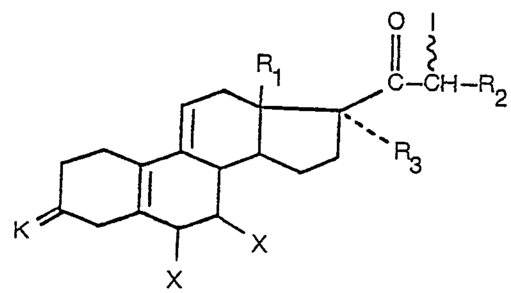

(IV)

C — the compounds with the formula (V):

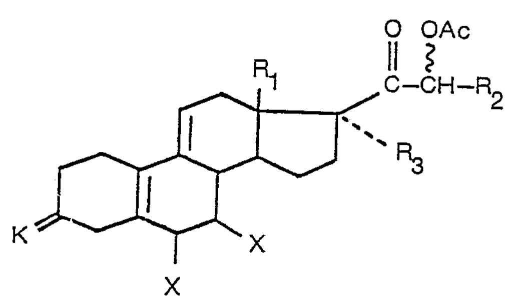

(V)

D — the compounds with the formula (VI):

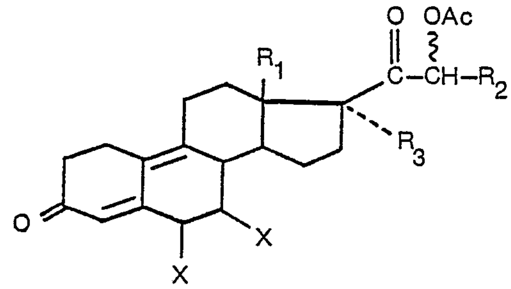

(VI)

in which formulae K represents a ketal group, X represents a hydrogen atom or a tritium atom, $R_1$ represents an alkyl radical containing 1—3 carbon atoms, $R_2$ represents an alkyl radical containing 1—12 carbon atoms, $R_3$ represents an alkyl radical containing 1—4 carbon atoms, in the form of each of the 21R or 21S epimers or in the form of a mixture of these epimers.

2. Compounds with the formula (III) according to Claim 1 which responds to the denomination 3-(-1,2-ethanediyl)-cyclo-acetal of 17$\alpha$-methyl-17$\beta$-(-2-hydroxy-1-oxopropyl)-estra-5 (10) 9 (11)-dien-3-one in the form of each of the epimers at the hydroxyl or in the form of a mixture of these epimers.

3. Compounds with the formula (IV) according to Claim 1 which respond to the denominations 3-(1,2-ethanediyl)-cyclo-acetal of 17$\alpha$-methyl-17$\beta$-(2-iodo-1-oxopropyl)-estra-5 (10) 9 (11)-dien-3-one and [-6,7$^3$H-]-3-(1,2-ethanediyl)-cyclo-acetal of 17$\alpha$-methyl-17$\beta$-(2-iodo-1-oxopropyl)-estra-5 (10) 9 (11)-dien-3-one in the form of each of the epimers at the iodine or in the form of a mixture of these epimers.

4. Compounds with the formula (V) according to Claim 1 responding to the denominations 3-(-1,2-ethanediyl)-cyclo-acetal of 17$\alpha$-methyl-17$\beta$-(-2-acetoxy-1-oxopropyl)-estra-5 (10) 9 (11)-dien-3-one and [-6,7$^3$H-]-3-(-1,2-ethanediyl)-cyclo-acetal of 17$\beta$-(-2-acetoxy-1-oxopropyl)-17$\alpha$-methyl-estra-5 (10) 9 (11)-dien-3-one, in the form of each of the epimers at the acetoxyl or in the form of a mixture of these epimers.

5. Compounds with the formula (VI) according to Claim 1 which respond to the denominations 17$\beta$-(-2-acetoxy-1-oxopropyl)-17$\alpha$-methylestra-4,9-dien-3-one and [-6,7$^3$H-]-17$\beta$-(-2-acetoxy-1-oxopropyl)-17$\alpha$-methylestra-4,9-dien-3-one, in the form of each of the epimers at the acetoxyl or in the form of a mixture of these epimers.

6. Preparation process for the compounds with the formula (III) as defined in Claim 1, characterized in that a compound with the formula (II):

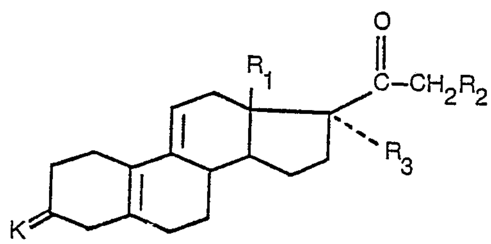

(II)

in which K represents a ketal group, and $R_1$, $R_2$ and $R_3$ retain the same significance as in Claim 1, is submitted to the action of an oxidation agent in the presence of a tertiary alcoholate, then to that of a reducing agent so as to obtain the compound with the formula (III) in the form of a mixture of 21R and 21S epimers.

7. Preparation process for the compounds with the formulae (IV), (V) and (VI) as defined in Claim 1, characterized in that a compound with the formula ($II_A$):

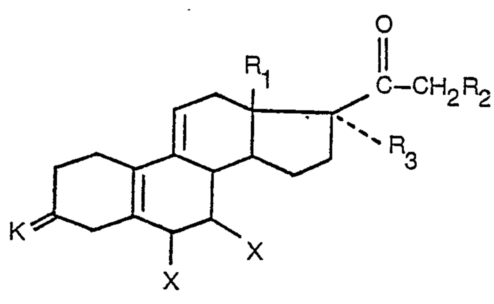

($II_A$)

in which K, X, $R_1$, $R_2$ and $R_3$ have the significance indicated in Claim 1, is submitted to the action of a strong base so as to form the intermediate enolate which is submitted to the action of iodine so as to obtain the compound with the formula (IV):

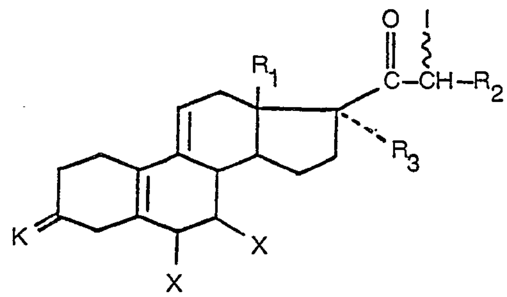

(IV)

in the form of a mixture of 21R and 21S isomers, which, if desired, is submitted to the action of an alkaline acetate, so as to obtain the compound with the formula (V):

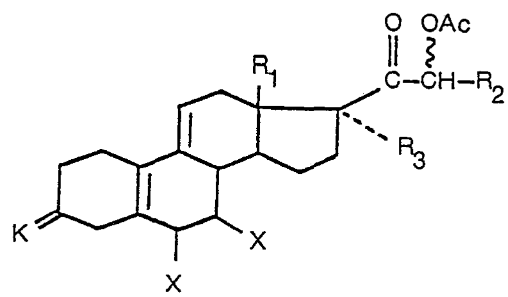

(V)

in the form of a mixture of 21R and 21S isomers, which, if desired, is submitted to the action of an acid hydrolyzing agent able to hydrolyze the ketal group and to isomerize the system of double bounds △ 5 (10) 9 (11) into a 4,9 system so as to obtain a compound with the formula (VI):

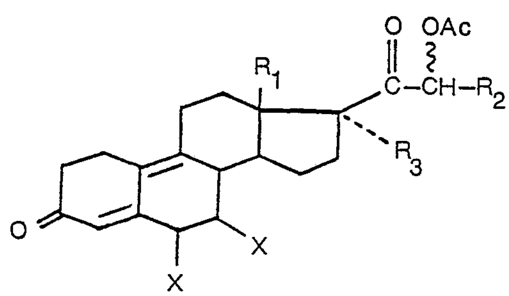

(VI)

in the form of a mixture of 21R and 21S isomers.

8. Use of the compounds with the formula (III) as defined in claim 1, in the preparation of compounds with the formula (I):

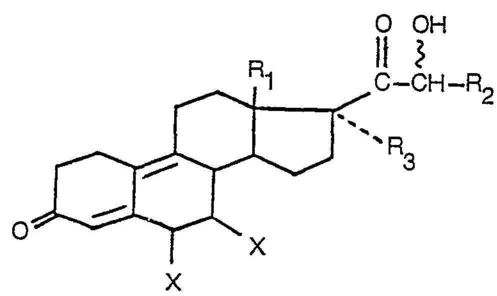

(I)

in which $R_1$, $R_2$ and $R_3$ have the significance indicated in Claims 1 and X represents a hydrogen atom, characterized in that the compound with the formula (III) is submitted to the action of an acid hydrolyzing agent able to hydrolyze the ketal group and to isomerize the system of double bonds 5 (10) 9 (11) into a Δ 4,9 system, so as to obtain the compound with the formula (I) in which X represents a hydrogen atom in the form of a mixture of 21R and 21S epimers which, if desired, is separated into each of the epimers.

9. Use of the compounds with the formula (VI) as defined in Claim 1 in the preparation of the compounds with the formula ($I_A$):

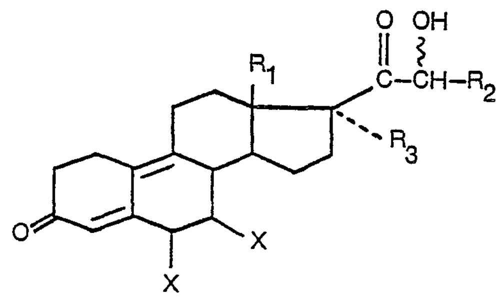

($I_A$)

in which X, $R_1$, $R_2$ and $R_3$ have the significance indicated in Claim 1, characterized in that a compound with the formula (VI) is submitted to the action of a saponification agent so as to obtain the compound with the formula ($I_A$) in the form of a mixture of 21R and 21S isomers which, if desired is separated into each of the epimers.